# EUROPEAN PATENT SPECIFICATION

(11) **EP 3 984 583 B1**
(45) Date of publication and mention of the grant of the patent: **29.10.2025**
(21) Application number: 21201412.0
(22) Date of filing: 07.10.2021
(51) Int. Cl.: A61M 25/02, A61J 15/00, A61F 13/0246, A61F 13/12

(54) **MEDICAL-TUBE AFFIXING DEVICE**
BEFESTIGUNGSVORRICHTUNG FÜR MEDIZINISCHEN SCHLAUCH
DISPOSITIF DE FIXATION DE TUBE MÉDICAL

(30) Priority: 12.10.2020 TW 109135090
(43) Date of publication of application: 20.04.2022
(73) Proprietor: Su, Chien-Chung, Taichung City 404 (TW)
(72) Inventor: Su, Chien-Chung, Taichung City 404 (TW)
(74) Representative: Viering, Jentschura & Partner mbB Patent- und Rechtsanwälte

(56) References cited:
- US-A- 5 188 609
- US-A- 5 944 696
- US-A1- 2009 306 603

## Description

### BACKGROUND OF THE INVENTION

### 1. Field of the Invention:

The present invention relates to affixion of medical tubes like nasogastric tubes or drainage tubes, and more particularly to a medical-tube affixing device that affixes a medical tube to a patient using the tube.

### 2. Description of the Related Art:

With the rapid progress of both science and medicine, there have been increasing improvements and breakthroughs in the field of detection and therapy of diseases, resulting in increased survival of people affected by physical injuries and diseases. Consequently, healthcare for such affected people have been receiving more and more attention. Medical tubes such as nasogastric tubes and drainage tubes are extensively used in various medical applications where feeding or drainage is needed. For example, people incapable of self-feeding due to physical conditions caused by injuries, diseases or aging, nasogastric tubes are usually used by medical staff or caregivers to provide feeding or pharmaceuticals, to inspect food remnant in stomach, and/or to draw gastric juice for examination.

In use, a nasogastric tube after properly placed in a patient's stomach leaves a free end outside the patient's nose to be further connected to a medical apparatus for guiding fluid in or out. Conventionally, since such a free end is not especially arranged and fixed, it tends to get caught unintentionally by surrounding objects when the patient turns his/her body or when a caregiver performs healthcare works, resulting in the patient's discomfort or even tube coming-off. This makes nasogastric tubes and other medical tubes a main source of inconvenience and health risks to patients.

For addressing the aforementioned concern, a common practice is to affix the free end of the tube to the patient's body using surgical tape. Specifically, the tape is wound around the tube and then adhered to the patient's nose bridge. For a coming feeding, the tape torn off from the patient's skin is usually discarded because it becomes less adhesive or broken, so the foregoing affixion work then has to be repeated every time the tube is used.

While this existing practice somehow works, it has obvious shortcomings:
1. The surgical tape has to be cut on site, adding operational inconvenience.
2. For better affixation, the surgical tape may press the tube against the patient's nose and nasal mucosa excessively, leading to nasal pressure ulcer and skin irritation.
3. Repeatedly tearing the tape off can pull the medical tube and/or hurt the patient's skin.
4. The existing surgical tape comes in rolls, so the non-adhesive surface may have glue reside, increasing the risks of contamination and infection.

US 5 188 609 A discloses a medical-tube affixing device, comprising an adhesive portion, a connecting portion and a clipping portion. The adhesive portion has an adhesive surface, a pivot post and a retaining top, and is configured to be reusably adhered to a patient's body. The pivot post and the retaining top are attached to a surface of the adhesive portion reverse to the adhesive surface. Similar medical-tube affixing devices are disclosed in US 2009/306603 A1 and US 5 944 696 A.

### SUMMARY OF THE INVENTION

To solve the foregoing problems, the present invention provides a medical-tube affixing device according to claim 1 or 8. Preferred embodiments are defined in the dependent claims.

The primary objective of the present invention is to provide a medical-tube affixing device that allows comfortable and adjustable affixation of a medical tube, thereby preventing pressure ulcer.

The secondary objective of the present invention is to provide a medical-tube affixing device that allows convenient affixation of a medical tube.

### BRIEF DESCRIPTION OF THE DRAWINGS

**Fig. 1** is a perspective view of the present invention.
**Fig. 2** is an exploded view of the present invention.
**Fig. 3** is a cross-sectional view of the present invention.
**Fig. 4** is another cross-sectional view of the present invention, illustrating its assembling.
**Fig. 5** is a further cross-sectional view of the present invention, illustrating its assembling.
**Fig. 6** is an applied view of the present invention, illustrating its installation.
**Fig. 7** is another applied view of the present invention, illustrating its adjustment.
**Fig. 8** is an applied perspective view of the present invention, illustrating its installation.
**Fig. 9** is another applied view of the present invention, illustrating its adjustment.
**Fig. 10** is a perspective view of another implementation of the present invention.
**Fig. 11** is a perspective view of a further implementation of the present invention.

### DETAILED DESCRIPTION OF THE INVENTION

While a preferred embodiment provided hereinafter for illustrating the concept of the present invention has been described above, it is to be understood that the components of the embodiment shown in the accompanying drawings are depicted with scale, dimensions and/or displacement facilitating easy explanation and need not to be made to exact scale.

As used herein, directional terms like "upper," "lower," "front," "rear," "left," "right," "inner," "outer," and "lateral" shall be defined with respect to the accompanying drawings. Therefore, theses directional terms are for illustration and understanding of the present invention, but not intended to limit the scope of the present invention.

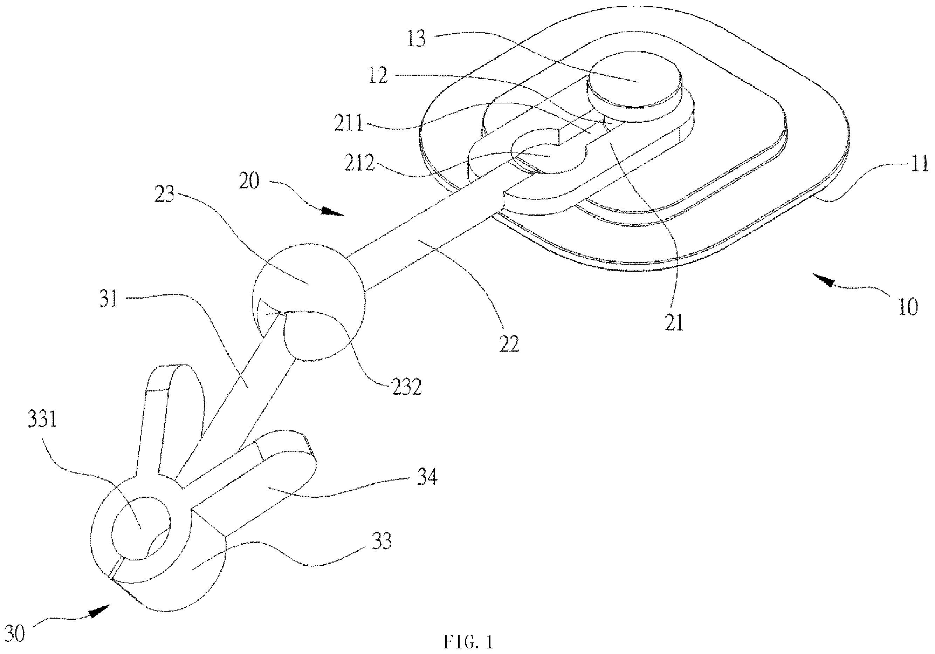

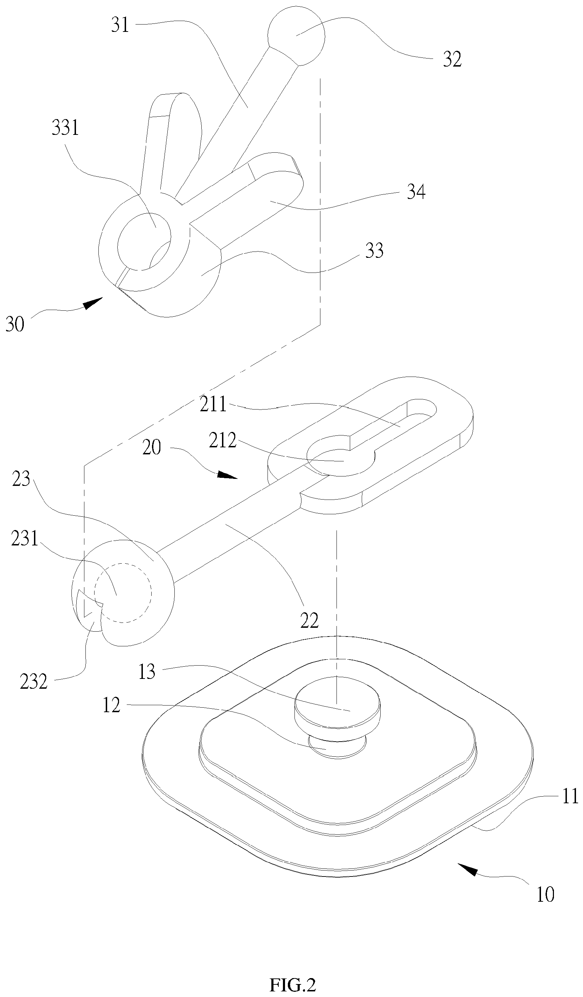

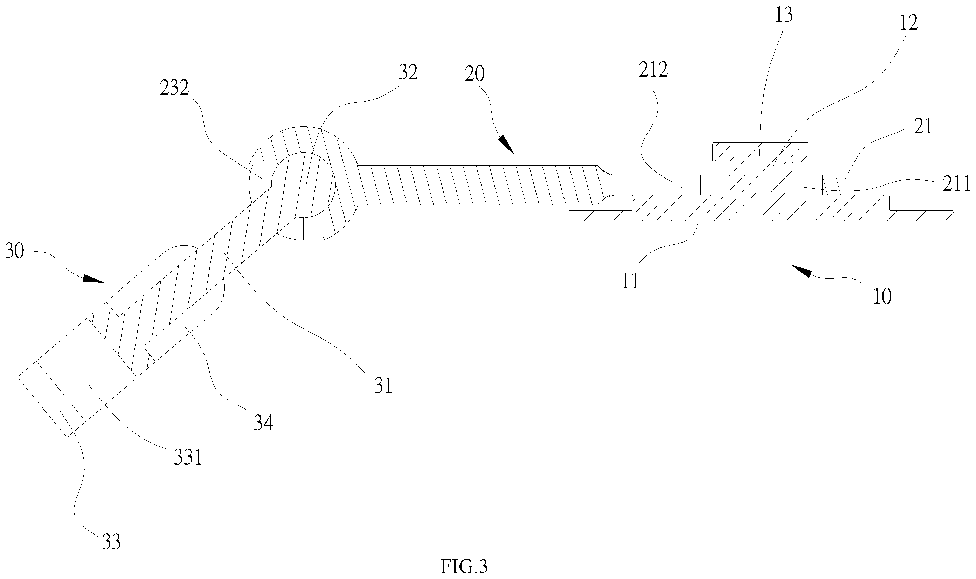

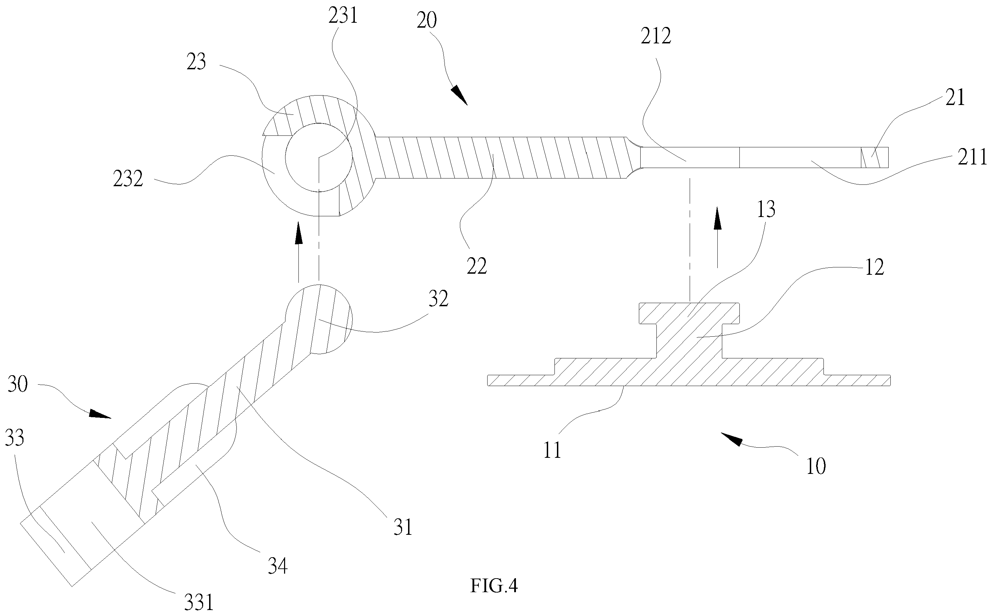

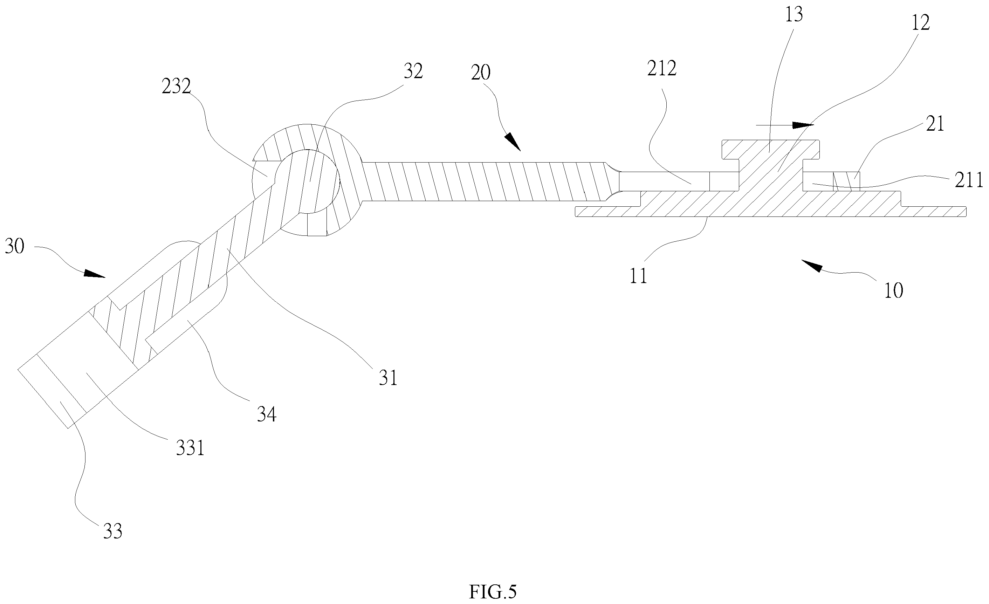

First, referring to **Fig. 1** through **Fig. 5****,** the present invention provides a medical-tube affixing device comprising an adhesive portion **10,** a connecting portion **20,** and a clipping portion **30.**

The adhesive portion **10** is a flat piece having an adhesive surface **11** made of a medically acceptable material, such as silicone. The adhesive surface **11** is configured to be reusably adhered to a patient's nose bridge. At the side of the adhesive portion **10** opposite to the adhesive surface **11,** there is a pivot post **12** and a retaining top **13** that are integratedly formed as a unity. The pivot post **12** has a diameter smaller than the diameter of the retaining top **13.**

The connecting portion **20** is a key-shaped member, having an elliptical yoke member **21,** an arm **22** and a ball-like socket **23** that are integratedly formed as a unity. The yoke member **21** is centrally formed with a slot **211.** The slot **211** has its end near the arm **22** formed as an assembling hole **212** that accommodates the retaining top **13** of the adhesive portion **10.** The slot **211** is configured to slidably receive the pivot post **12** of the adhesive portion **10.** The arm **22** has one end connected to the end of the yoke member **21** having the assembling hole **212** and an opposite end connected to the ball-like socket **23.** The ball-like socket **23** defines therein a round chamber **231.** The chamber **231** is communicated with the exterior through a notch **232** formed at a free end of the ball-like socket **23.**

The clipping portion **30** is a pendulum-like structure connected to the connecting portion **20.** The clipping portion **30** has a link **31** that has a round cross-section and is terminated with a ball-like end **32.** The ball-like end **32** is configured to be pivotally received in the chamber **231** of the ball-like socket **23.** The link **31** has a pair of jaws **33** defining an accommodating space **331** at the end opposite to the ball-like end **32.** The accommodating space **331** defined by the closed jaws **33** is not shaped as a true round for better holding medical tubes that are only made with round cross-sections. The jaws **33** are configured to be operated to open or close by two elliptical handles **34.** In the clipping portion **30,** the link **31,** the jaws **33,** and the handles **34** are integratedly formed as a unity.

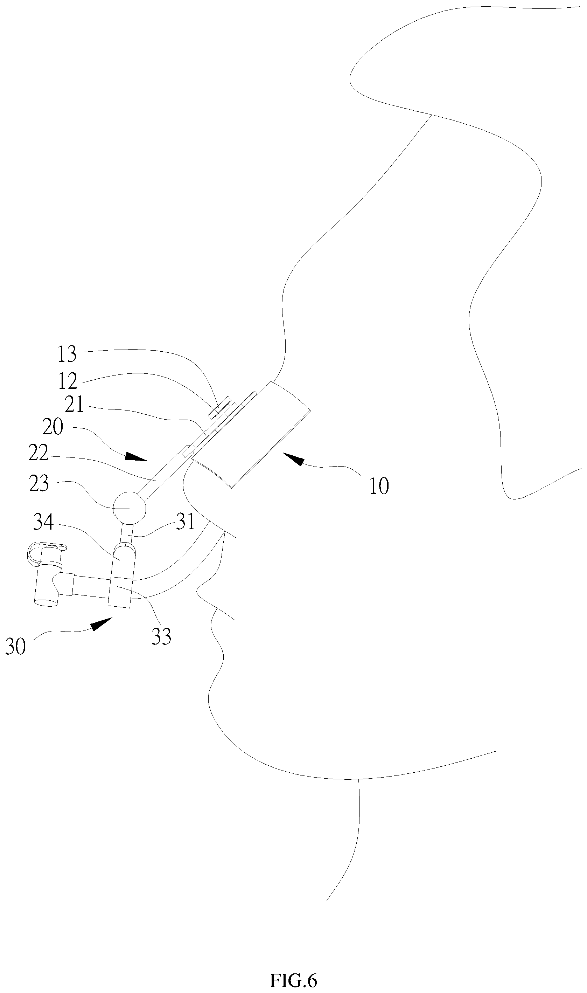

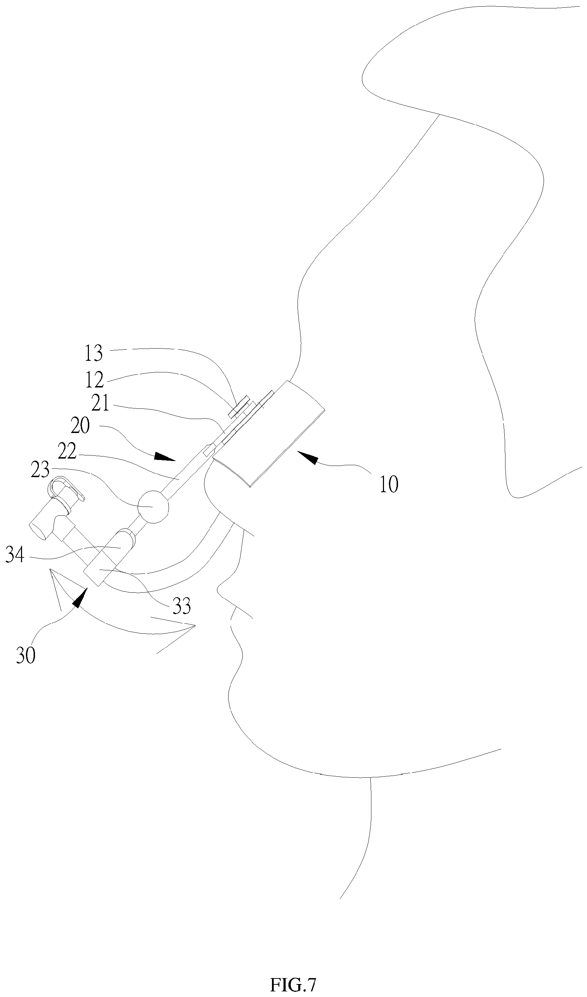

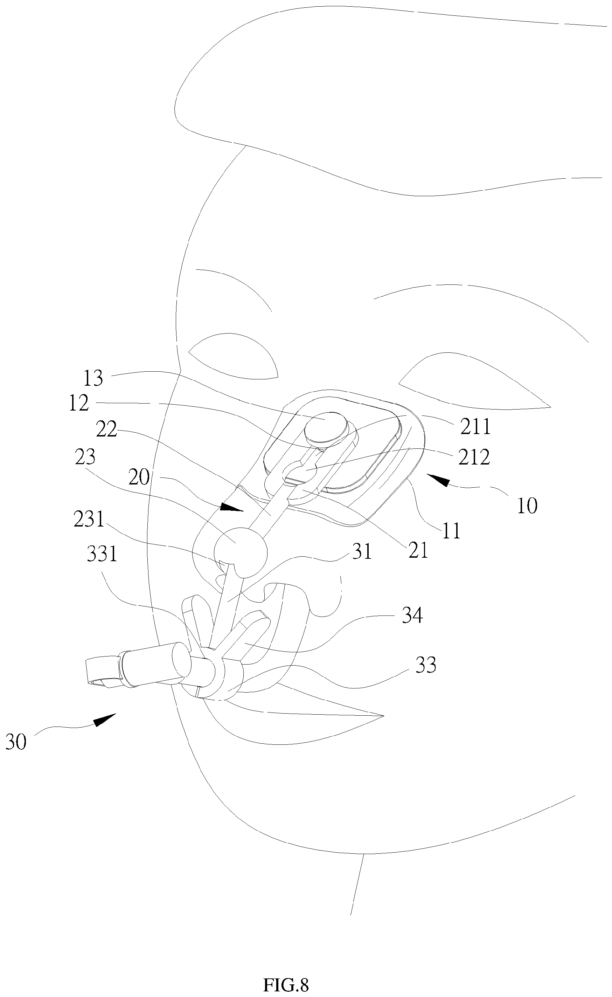

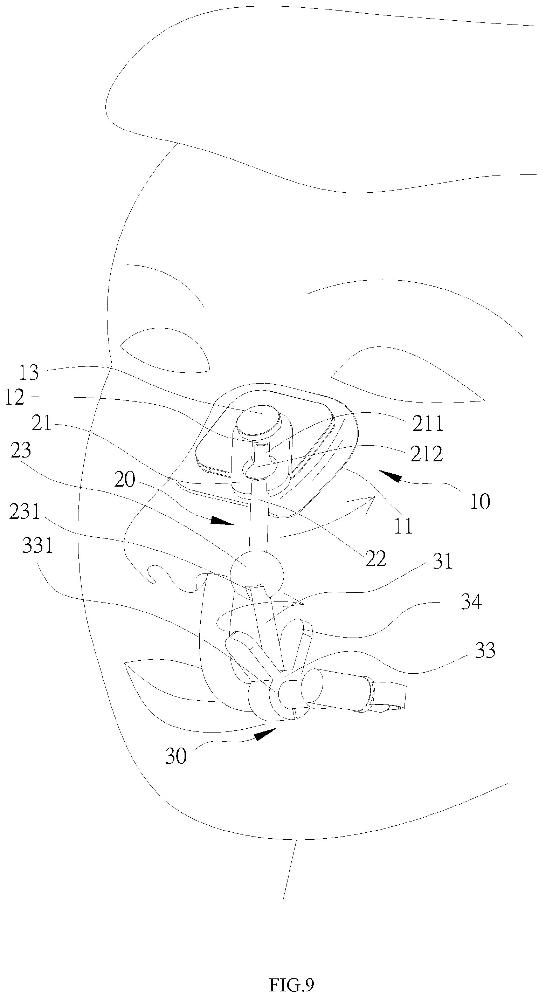

In use of the disclosed medical-tube affixing device, referring to **Fig. 6** through **Fig. 9****,** the silicone-made adhesive surface **11** of the adhesive portion **10** is put on the patient's nose bridge. Then the yoke member **21** can be attached to the pivot post **12** by aligning the assembling hole **212** with the retaining top **13** and sliding the pivot post **12** along the slot **211** communicated with the assembling hole **212,** so that the yoke member **21** is retained on the adhesive portion **10** by the retaining top **13.** The chamber **231** of the ball-like socket **23** of the connecting portion **20** pivotally receives the ball-like end **32** of the clipping portion **30.** When an operator uses his/her thumb and forefinger to draw the two handles **34** together toward the link **31,** the two jaws **33** operatively connected with the handles **34** are separated. Then a nasogastric tube can be put into an accommodating space **331** defined between the jaws **33** and when the handles **34** are released by the operator, the nasogastric tube is held in position in the patient's nostril. With the ball-like end **32** and the link **31** of the clipping portion **30,** the nasogastric tube is allowed to be positioned and posed for both the greatest comfort of the patient and the best efficiency of tube feeding.

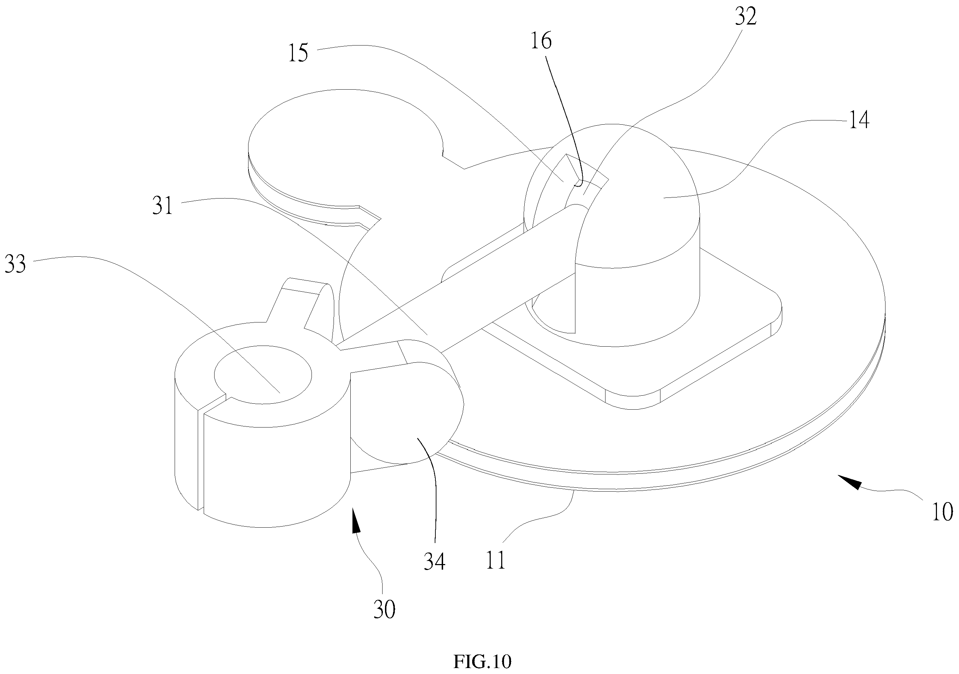

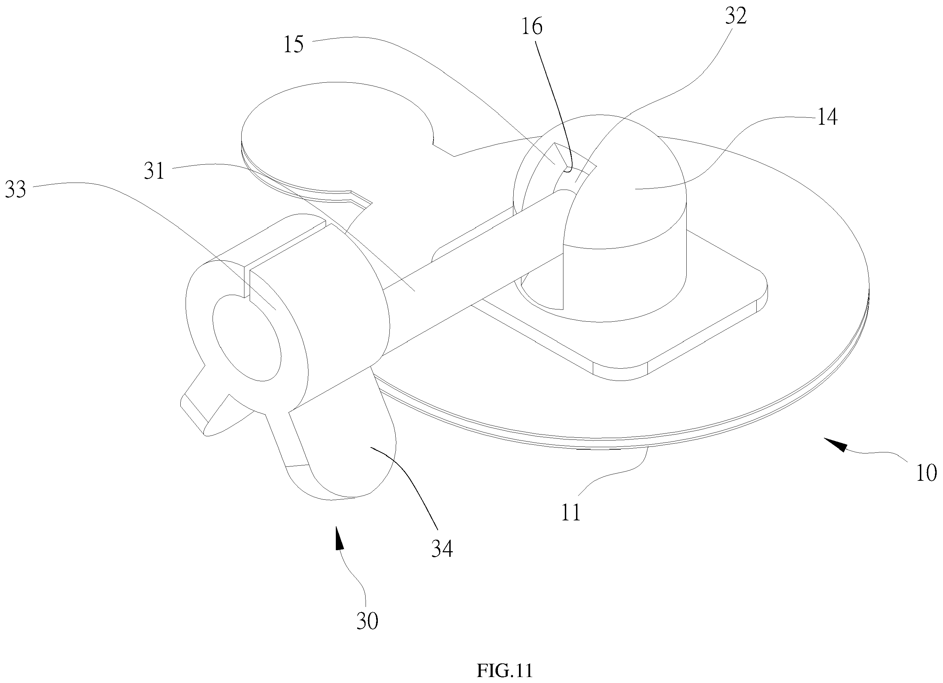

Referring to **Fig. 10** and **Fig. 11****,** in another implementation of the present, the disclosed affixing device may be implemented for holding a drainage tube in position around a patient's chest or abdomen for postoperative care. Therein, the affixing device comprises, from bottom to top as shown, an adhesive portion **10;** a pivot base **14** raised from the adhesive portion **10** and laterally formed with a notch **15,** which is communicated with a round chamber **16** for rotatably receiving a ball-like end **32** of a clipping portion **30,** such that the ball-like end **32** when installed can rotate with respect to the adhesive portion **10.** The side of the adhesive portion **10** reverse to the pivot base **14** is formed with an adhesive surface **11** that is configured to be adhesively affixed to a patient's chest or abdomen. The clipping portion **30** has, in addition to the ball-like end **32,** a link **31** that connects the ball-like end **32** with two handles **34.** When an operator uses his/her thumb and forefinger to draw the two handles **34** together toward the link **31,** two jaws **33** operatively connected with the handles **34** are separated. At this time, a drainage tube can be put into an accommodating space **331** defined between the jaws **33** and when the handles **34** are released by the operator, the drainage tube is adjustably held in position around the patient's chest or abdomen. With the ball-like end **32** and the link **31** of the clipping portion **30,** the drainage tube is allowed to be positioned and posed for both the greatest comfort of the patient and the best efficiency of the intended drainage.

With the configurations described above with reference to the particular embodiments, the present invention provides the following beneficial effects:
1. The adhesive portion **10,** the connecting portion **20,** and the clipping portion **30** work together to provide convenient affixion of a medical tube.
2. The ball-like end **32** and the link **31** of the clipping portion 30 allows free adjustment of the installed medical tube, thereby ensuring the patient's comfort.
3. The medical-tube affixing device of the present invention achieves reliable affixion while preventing form putting excessive pressure on the patient's nasal mucosa and skin.

The present invention has been described with reference to the preferred embodiments and it is understood that the embodiments are not intended to limit the scope of the present invention, which is defined by the appended claims.

## Claims

1. A medical-tube affixing device, comprising:
an adhesive portion (10), having an adhesive surface (11), a pivot post (12), and a retaining top (13); the adhesive surface (11) being configured to be reusably adhered to a patient's nose bridge; the pivot post (12) and the retaining top (13) being integratedly formed as a unity and attached to a surface of the adhesive portion (10) reverse to the adhesive surface (11); the pivot post (12) having a diameter smaller than a diameter of the retaining top (13);
a connecting portion (20) , having a yoke member (21), an arm (22), and a ball-like socket (23); the yoke member (21) being centrally formed with a slot (211); the slot (211) having an end near the arm (22) formed as an assembling hole (212) that accommodates the retaining top (13) of the adhesive portion (10); the slot (211) being configured to slidably receive the pivot post (12) of the adhesive portion (10); the arm (22) having one end connected to the end of the yoke member (21) and an opposite end connected to the ball-like socket (23); the ball-like socket (23) defining therein a chamber (231); and the chamber (231) being communicated with the exterior through a notch (232) formed at a free end of the ball-like socket (23); and
a clipping portion (30), having a link (31) that is terminated with a ball-like end (32); the ball-like end (32) being configured to be pivotally received in the chamber (231) of the ball-like socket (23); the link (31) having a pair of jaws (33) defining an accommodating space (331) at the end opposite to the ball-like end (32); and the jaws (33) being configured to be operated to open or close by two handles (34).

2. The medical-tube affixing device of claim 1, wherein the adhesive portion (10) is made of a medically acceptable material, like silicone.

3. The medical-tube affixing device of claim 1, wherein the connecting portion (20) is a key-shaped member, and the yoke member (21) of the connecting portion (20) is elliptical in shape, in which the yoke member (21), the arm (22) and the ball-like socket (23) are integratedly formed as a unity.

4. The medical-tube affixing device of claim 1, wherein the clipping portion (30) is a pendulum-like structure.

5. The medical-tube affixing device of claim 1, wherein in the clipping portion (30), the link (31), the jaws (33), and the handles (34) are integratedly formed as a unity.

6. The medical-tube affixing device of claim 1, wherein the link (31) of the clipping portion (30) has a round cross-section, and the accommodating space (331) defined by the closed jaws (33) is not shaped as a true round.

7. The medical-tube affixing device of claim 1, wherein the handles (34) are each an elliptical piece.

8. A medical-tube affixing device, comprising:
an adhesive portion (10), having a pivot base (14) raised therefrom; the pivot base (14) being laterally formed with a notch (15) which is communicated with a round chamber (16), the adhesive portion (10) having adhesive surface (11) that is reverse to pivot base (14) and is configured to be adhesively affixed to a patient's body; and
a clipping portion (30), having a link (31) and a pair of jaws (33); the link (31) having a ball-like end (32) configured to be pivotally received in the round chamber (16), so that the ball-like end (32) is allowed to rotate in the round chamber (16) with respect to the adhesive portion (10); and the clipping portion (30) having two handles (34) operatively connected to the jaws (33), so that when the two handles (34) are drawn together, the jaws (33) are opened for a medical tube to be placed into an accommodating space (331) defined between the jaws (33), and when the handles (34) are released, the medical tube is hold by the jaws (33), wherein the ball-like end (32) and the link (31) of the clipping portion (30) work together to allow the medical tube to be adjustably held in position with respect to the patient's body.

## Patentansprüche

1. Ein Befestigungsgerät für medizinische Schläuche, umfassend:
einen Klebeabschnitt (10), der eine Klebefläche (11), einen Drehstab (12) und einen Halteaufsatz (13) aufweist; wobei die Klebefläche (11) so konfiguriert ist, dass sie wiederverwendbar auf den Nasenrücken eines Patienten geklebt werden kann; wobei der Drehstab (12) und der Halteaufsatz (13) einstückig ausgebildet und auf einer der Klebefläche (11) gegenüberliegenden Oberfläche des Klebeabschnitts (10) befestigt sind; wobei der Durchmesser des Drehstabs (12) kleiner als der Durchmesser des Halteaufsatzes (13) ist;
einen Verbindungsabschnitt (20), der ein Gabelelement (21), einen Arm (22) und eine kugelförmige Aufnahmebuchse (23) aufweist; wobei das Gabelelement (21) mittig mit einem Schlitz (211) ausgebildet ist; das in der Nähe des Arms (22) befindliche Ende des Schlitzes (211) als Montageloch (212) ausgebildet ist, welches den Halteaufsatz (13) des Klebeabschnitts (10) aufnimmt; der Schlitz (211) so konfiguriert ist, dass er den Drehstab (12) des Klebeabschnitts (10) verschiebbar aufnimmt; der Arm (22) mit einem Ende mit dem Gabelelement (21) und mit dem anderen Ende mit der kugelförmigen Aufnahmebuchse (23) verbunden ist; die kugelförmige Aufnahmebuchse (23) in ihrem Inneren eine Kammer (231) aufweist; die Kammer (231) über eine an einem freien Ende der kugelförmigen Aufnahmebuchse (23) ausgebildete Ausnehmung (232) mit dem Außenbereich in Verbindung steht;
und
einen Klemmabschnitt (30), der ein Verbindungstück (31) aufweist, das mit einem kugelförmigen Ende (32) abschließt; wobei das kugelförmige Ende (32) so konfiguriert ist, dass es schwenkbar in der Kammer (231) der kugelförmigen Aufnahmebuchse (23) aufgenommen ist; das Verbindungstück (31) ein Paar Klemmbacken (33), die an dem dem kugelförmigen Ende (32) gegenüberliegenden Ende einen Aufnahmeraum (331) bilden, aufweist; und die Klemmbacken (33) so konfiguriert sind, dass sie durch zwei Griffe (34) zum Öffnen und Schließen betätigbar sind.

2. Befestigungsgerät für medizinische Schläuche nach Anspruch 1, bei dem der Klebeabschnitt (10) aus einem medizinisch verträglichen Material wie Silikon besteht.

3. Befestigungsgerät für medizinische Schläuche nach Anspruch 1, bei dem der Verbindungsabschnitt (20) ein schlüsselförmiges Element ist und das Gabelelement (21) des Verbindungsabschnitts (20) elliptisch geformt ist, wobei das Gabelelement (21), der Arm (22) und die kugelförmige Aufnahmebuchse (23) einstückig ausgebildet sind.

4. Befestigungsgerät für medizinische Schläuche nach Anspruch 1, bei dem der Klemmabschnitt (30) eine pendelartige Struktur ist.

5. Befestigungsgerät für medizinische Schläuche nach Anspruch 1, bei dem der Klemmabschnitt (30), das Verbindungstück (31), die Klemmbacken (33) und die Griffe (34) einstückig ausgebildet sind.

6. Befestigungsgerät für medizinische Schläuche nach Anspruch 1, bei dem das Verbindungstück (31) des Klemmabschnitts (30) einen runden Querschnitt aufweist und der durch die geschlossenen Klemmbacken (33) definierte Aufnahmeraum (331) nicht kreisrund ausgebildet ist.

7. Befestigungsgerät für medizinische Schläuche nach Anspruch 1, bei dem die Griffe (34) jeweils ein elliptisches Teil sind.

8. Ein Befestigungsgerät für medizinische Schläuche, umfassend:
einen Klebeabschnitt (10), der eine von ihm vorstehende Schwenkbasis (14) aufweist; wobei die Schwenkbasis (14) seitlich mit einer Ausnehmung (15) ausgebildet ist, welche mit einer runden Kammer (16) in Verbindung steht; wobei der Klebeabschnitt (10) auf der der Schwenkbasis (14) gegenüberliegenden Seite mit einer Klebefläche (11) versehen ist, die zur adhäsiven Befestigung am Körper eines Patienten vorgesehen ist; und
einen Klemmabschnitt (30), der ein Verbindungstück (31) und ein Paar Klemmbacken (33) aufweist; wobei das Verbindungstück (31) ein kugelförmiges Ende (32) aufweist, das so konfiguriert ist, dass es schwenkbar in der runden Kammer (16) aufgenommen ist, sodass das kugelförmige Ende (32) in der runden Kammer (16) relativ zum Klebeabschnitt (10) drehbar ist; und der Klemmabschnitt (30) zwei Griffe (34) aufweist, die wirkend mit den Backen (33) verbunden sind, sodass, wenn die beiden Griffe (34) zusammengeführt werden, die Backen (33) geöffnet werden, damit ein medizinischer Schlauch in einen zwischen den Backen (33) gebildeten Aufnahmeraum (331) gelegt werden kann, und wenn die Griffe (34) losgelassen werden, der medizinische Schlauch von den Backen (33) gehalten wird, wobei das kugelartige Ende (32) und das Verbindungsstück (31) des Klemmabschnitts (30) zusammenwirken, um zu ermöglichen, dass der medizinische Schlauch in Bezug auf den Körper des Patienten einstellbar in Position gehalten wird.

## Revendications

1. - Dispositif de fixation d'un tube médical, comprenant :
une partie adhésive (10), ayant une surface adhésive (11), un axe de pivot (12), et une partie supérieure de retenue (13) ; la surface adhésive (11) étant configurée pour être collée de manière réutilisable à l'arête du nez d'un patient ; l'axe de pivot (12) et la partie supérieure de retenue (13) étant formés de manière intégrée en tant qu'unité et attachés à une surface de la partie adhésive (10) au dos de la surface adhésive (11), l'axe de pivot (12) ayant un diamètre inférieur à un diamètre de la partie supérieure de retenue (13) ;
une partie de laison (20), comportant un élément étrier (21), un bras (22) et une douille en forme de boule (23), l'élément étrier (21) comportant au centre une fente (211), la fente (211) ayant une extrémité proche du bras (22) formée en tant que trou d'assemblage (212) qui reçoit la partie supérieure de retenue (13) de la partie adhésive (10), la fente (211) étant configurée pour recevoir de manière coulissante l'axe de pivot (12) de la partie adhésive (10), le bras (22) ayant une extrémité reliée à l'extrémité de l'élément étrier (21) et une extrémité opposée reliée à la douille en forme de boule (23), la douille en forme de boule (23) définissant à l'intérieur une chambre (231), et la chambre (231) étant en communication avec l'extérieur à travers une encoche (232) formée à une extrémité libre de la douille en forme de boule (23) ; et
une partie de clipsage (30), ayant une liaison (31) qui est terminée par une extrémité en forme de boule (32), l'extrémité en forme de boule (32) étant configurée pour être reçue de manière pivotante dans la chambre (231) de la douille en forme de boule (23), la liaison (31) ayant une paire de mâchoires (33) définissant un espace de réception (331) à l'extrémité opposée à l'extrémité en forme de boule (32), et les mâchoires (33) étant configurées pour être actionnées pour s'ouvrir ou se fermer par deux branches (34).

2. - Dispositif de fixation d'un tube médical selon la revendication 1, dans lequel la partie adhésive (10) est faite d'un matériau médicalement acceptable, comme le silicone.

3. - Dispositif de fixation d'un tube médical selon la revendication 1, dans lequel la partie de liaison (20) est un élément en forme de clé, et l'élément étrier (21) de la partie de liaison (20) est de forme elliptique, l'élément étrier (21), le bras (22) et la douille en forme de boule (23) étant formés de manière intégrée en tant qu'unité.

4. - Dispositif de fixation d'un tube médical selon la revendication 1, dans lequel la partie de clipsage (30) est une structure de type pendule.

5. - Dispositif de fixation d'un tube médical selon la revendication 1, dans lequel la partie de clipsage (30), la liaison (31), les mâchoires (33) et les branches (34) sont formés de manière intégrée en tant qu'unité.

6. - Dispositif de fixation d'un tube médical selon la revendication 1, dans lequel la liaison (31) de la partie de clipsage (30) a une section transversale arrondie, et l'espace de réception (331) défini par les mâchoires fermées (22) n'a pas la forme d'un vrai rond.

7. - Dispositif de fixation d'un tube médical selon la revendication 1, dans lequel les branches (34) sont chacune une pièce elliptique.

8. - Dispositif de fixation d'un tube médical, comprenant :
une partie adhésive (10), ayant une base pivot (14) surélevée par rapport à celle-ci, la base pivot (14) comportant latéralement avec une encoche (15) qui est en communication avec une chambre arrondie (16), la partie adhésive (10) ayant une surface adhésive (11) qui est au dos de la base pivot (14) et est configurée pour être fixée de manière adhésive au corps d'un patient ; et
une partie de clipsage (30), ayant une liaison (31) et une paire de mâchoires (33), la liaison (31) ayant une extrémité en forme de boule (32) configurée pour être reçue de manière pivotante dans la chambre arrondie (16), de telle sorte que l'extrémité en forme de boule (32) est amenée à tourner dans la chambre arrondie (16) par rapport à la partie adhésive (10), et la partie de clipsage (30) ayant deux branches (34) reliées de manière fonctionnelle aux mâchoires (33), de telle sorte que, lorsque les deux branches (34) sont rapprochées, les mâchoires (33) s'ouvrent pour qu'un tube médical puisse être placé dans un espace de réception (331) défini entre les mâchoires (33), et lorsque les branches (34) sont relâchées, le tube médical est maintenu par les mâchoires (33), l'extrémité en forme de boule (32) et la liaison (31) de la partie de clipsage (30) coopérant pour permettre au tube médical d'être maintenu en position de manière ajustable par rapport au corps du patient.
